**Europäisches Patentamt**

**European Patent Office**

**Office européen des brevets**

(19)

(11) Veröffentlichungsnummer: **0 385 250**
**A2**

(12) ## EUROPÄISCHE PATENTANMELDUNG

(21) Anmeldenummer: **90103322.5**

(22) Anmeldetag: **21.02.90**

(51) Int. Cl.5: **A61M 15/00**

(30) Priorität: **28.02.89 DE 3906202**

(43) Veröffentlichungstag der Anmeldung:
**05.09.90 Patentblatt 90/36**

(84) Benannte Vertragsstaaten:
**DE FR GB IT**

(71) Anmelder: **MEDICOMMERZ GMBH**
**Am Schmiedacker 11**
**D-7815 Kirchzarten(DE)**

(72) Erfinder: **Glitsch, Hans W.**
**Am Schmiedacker 11**
**D-7815 Kirchzarten(DE)**

(74) Vertreter: **Schmitt, Hans, Dipl.-Ing. et al**
**Patentanwälte Dipl.-Ing H. Schmitt Dipl.-Ing.**
**W. Maucher Dreikönigstrasse 13**
**D-7800 Freiburg(DE)**

(54) **Verfahren und Vorrichtung zur Zuführung von Atemgas.**

(57) Die Erfindung betrifft ein Verfahren zur Zuführung von zusätzlichem Atemgas von einer Atemgasquelle zu einem spontan atmenden Patienten,wobei
das Atemgas dem Patienten während des Einatmens
(Inspirationsphase eines Atemzyklus) zugeführt wird.
Um bei dieser intermittierenden Atemgaszufuhr dem
Patienten das Atemgas so zuführen zu können, daß
dieser die Gaszufuhr praktisch nicht als störend
empfindet, ist erfindungsgemäß vorgesehen, daß der
Verlauf des pro Zeiteinheit dem Patienten zugeführten Atemgas-Volumens an den Verlauf des pro Zeiteinheit vom Patienten eingeatmeten Luftvolumens
über der Zeit zumindest in einem Teilbereich der
Inspirationsphase etwa angepaßt wird. Die Erfindung
betrifft auch eine Vorrichtung zur Zuführung von
Atemgas zu einem Patienten mit einer Atemgasquelle, einer Atemgaszuführeinrichtung, einem
Inhalations-oder dergleichen Atemsensor (10), einer
Meßeinrichtung sowie zumindest einem, mit dem
Atemsensor (10) und der Meßeinrichtung in Steuerverbindung stehenden Ventil. Insbesondere zur
Durchführung des eingangs erwähnten Verfahrens
ist (sind) das Ventil (die Ventile) als Dosierventil (1,
19) ausgebildet, welches (welche) zusammen mit
dem Atemsonsor (10) an eine die Atemgaszufuhr
während der Inspirationsphase eines Atemzyklus regelnde Steuereinrichtung (9, 17) angeschlossen ist
(sind).

Fig. 6

## Verfahren und Vorrichtung zur Zuführung von Atemgas

Die Erfindung betrifft ein Verfahren zur Zuführung von mit Sauerstoff angereichertem, zusätzlichem Atemgas von einer Atemgasquelle zu einem spontan atmenden Patienten, wobei das Atemgas dem Patienten während der eigenen Aktivierung seiner Lunge beim Einatmen (Inspirationsphase eines Atemzyklus) zugeführt wird. Die Erfindung betrifft auch eine Vorrichtung zur Zuführung von zusätzlichem Atemgas zu einem spontan bzw. selbst atmenden Patienten, insbesondere zur Durchführung des eingangs erwähnten Verfahrens, mit einer Atemgasquelle, einer Atemgaszuführeinrichtung, einem Inhalations- oder dergleichen Atemsensor, einer Meßeinrichtung sowie zumindest einem, mit dem Atemsensor und der Meßeinrichtung in Steuerverbindung sehenden Ventil.

Man kennt bereits verschiedene Verfahren und Vorrichtungen, um beispielsweise bei chronischen Hypoxämien die Atemluft eines selbst atmenden Patienten mit zusätzlichem Sauerstoff anreichern zu können.

So hat man bereits eine Vorrichtung geschaffen, die eine an eine Atemgasquelle angeschlossene Atemgaszuführeinrichtung mit zwei Nasenröhrchen aufweist (vgl.DE-OS 37 08 146).

Diese Nasenröhrchen sind jeweils in die Nasenlöcher eines Patienten einsetzbar. Während der Inspirationsphase eines Atemzyklus atmet der Patient somit nicht nur die Atemluft seiner Umgebung, sondern zusätzlich auch das von der Atemgasquelle über die Nasenröhrchen in seine Nasenlöcher einströmende Atemgas ein. Dabei ist an einem der Nasenröhrchen eine, mit einer Meßeinrichtung verbundene Inhalationssonde als Atemsensor vorgesehen und beispielsweise im Wege des beim Ausatmen abgegebenen, verbrauchten Atemgases angeordnet. Mit Hilfe des Atemsensors sowie einer damit in Steuerverbindung stehenden Meßeinrichtung zum Erfassen des Wechsels zwischen Inspirationsphase und Exspirationsphase wird ein in der Atemgaszuführung befindliches Ventil jeweils in der Exspirationsphase eines Atemzyklus geschlossen und während den jeweils nachfolgenden Inspirationsphasen wieder geöffnet.

Bei dieser vorbekannten Vorrichtung wird das Atemgas somit pulsierend jeweils in Atemgasstössen oder -schüben während den Inspirationsphasen abgegeben. Somit können in vorteilhafter Weise erhebliche Mengen von Atemgas eingespart werden, das beispielsweise in den Exspirationsphasen, also während des Ausatmens, vom Patienten ohnehin kaum aufgenommen würde. Dabei wird das aus dem Atmungsvorgang gewonnene Signal zum An- und Abschalten der Gasströmung benützt und die Abgabe des Atemgases an den Patienten auf die Einatmungsphase begrenzt.

Mit einer solchen Impulstechnik versucht man, die Sauerstoffversorgung vom Patienten beispielsweise mit kleineren Gasgeneratoren in gleicher Weise zu sichern, wie mit größeren, teuren und energieaufwendigeren Generatoren. Geräte mit vergleichsweise geringer Baugröße sowie entsprechend geringer Wärme- und Geräuschentwicklung sind vor allem in der Sauerstoff-Langzeit-Heimtherapie erwünscht.

Problematisch bei einer solchen intermittierenden Zuführung von Atemgas ist jedoch, daß die Atemgaszufuhr vom Patienten nur bis zu einer bestimmten Strömungsgeschwindigkeit toleriert wird; das Einsetzen der Gaszufuhr mit einer höheren Strahlgeschwindigkeit wird von manchen Patienten häufig als zu starker Gasstoß empfunden.

Es besteht daher die Aufgabe, ein Verfahren sowie eine Vorrichtung der eingangs erwähnten Art zu schaffen, mit deren Hilfe dem Patienten Atemgas zugeführt werden kann, praktisch ohne daß dieser die Atemgaszufuhr als störend empfindet. Dabei soll dem Patienten einerseits möglichst ausreichend Atemgas zugeführt werden können, andererseits aber auch das Atemgas weitgehend ohne überschüssige und vom Patienten zu diesem Zeitpunkt nicht aufnehmbare Atemgasmengen abgegeben werden.

Die erfindungsgemäße Lösung dieser scheinbar widersprüchlichen Aufgabe besteht bei dem Verfahren der eingangs erwähnten Art insbesondere darin, daß der Verlauf des pro Zeiteinheit dem Patienten zugeführten Atemgas-Volumens an den Verlauf des pro Zeiteinheit vom Patienten eingeatmeten Luftvolumens über der Zeit zumindest in einem Teilbereich der Inspirationsphase etwa angepaßt wird.

Mit dieser Anpassung der Atemgas-und Luftvolumina über der Zeit ist gleichzeitig auch eine Anpassung der Strömungsgeschwindigkeiten der Atemgaszufuhr und der vom Patienten selbst eingeatmeten Luft über der Zeit verbunden.

Bei dem erfindungsgemäßen Verfahren wird also die Volumenleistung (V : t) der Atemgaszufuhr an die Volumenleistung V : t) der vom Patienten selbst eingeatmeten Luft über der Zeit während der Inspirationsphase etwa angepaßt. Im Gegensatz zu einer intermittierenden Atemgaszufuhr, bei der dem Patienten während den Inspirationsphasen jeweils Atemgasstösse oder -schübe etwa gleichen Volumens pro Zeiteinheit zugeführt werden, kann bei dem erfindungsgemäßen Verfahren das dem Patienten pro Zeiteinheit zugeführte Atemgasvolumen so eingeregelt werden, daß dieses nicht das Luftvolumen übersteigt, das vom Patienten normalerweise spontan in derselben Zeiteinheit eingeatmet würde. Auf diese Weise wird nicht nur eine übermäßige Atemgaszufuhr vermieden, vielmehr kann etwa durch das Ansteigen der Atemgaszufuhr in dem Anfangsbereich der Inspirationsphase entsprechend dem dortigen Verlauf des vom Patienten pro Zeiteinheit eingeatmeten Luftvolumens ein plötzlich einsetzender Atemgasstoß oder -schub vermieden werden, der vom Patienten eventuell als störend empfunden würde.

Um im Bedarfsfall auch Patienten mit einem hohen Sauerstoff-Bedarf ausreichend mit Atemgas versorgen zu können, ist es vorteilhaft, wenn das pro Zeiteinheit zugeführte Atemgas-Volumen über der Zeit während der Inspirationsphase auf etwa die Werte des vom Patienten eingeatmeten Luft-Volumens über der Zeit eingeregelt wird.

Nach einem besonders vorteilhaften Vorschlag gemäß der Erfindung ist vorgesehen, daß der Startpunkt der von dem Beginn der Inspirationsphase ausgelösten Atemgaszufuhr eingestellt und vorzugsweise jeweils an dem Beginn der Inspirationsphase angenähert wird.

Da der Startpunkt der Atemgaszufuhr jeweils an dem Beginn der Inspirationsphase angenähert wird, erhält der Patient weitgehend bereits zu Beginn des Einatmungsvorganges ausreichend Atemgas zugeführt. Mit der Annäherung des Startpunktes der Atemgaszufuhr an den Beginn der Inspirationsphase kann bei der intermittierenden Gaszufuhr vergleichsweise viel Gas eingespart werden.

Dabei sieht eine Weiterbildung gemäß der Erfindung vor, daß von einer Steuereinrichtung aus den von einem Inhalations-od. dgl. Atemsensor kommenden Signalen der Abstand zwischen den Inspirationsphasen der Atemzyklen errechnet und mit Hilfe dieses Rechenergebnisses der Startpunkt der Atemgasabgaben vorzugsweise jeweils dem Beginn der Inhalationsphasen angenähert wird. Mit Hilfe der Steuereinrichtung kann aus mehreren Atemzügen der Abstand zwischen den Atemzyklen elektronisch errechnet und die Gaszufuhr auch in einer beliebigen Phase des Atemzyklus ausgelöst werden. Gleichzeitig ist es aber auch möglich, die Atemgaszufuhr um eine beliebige Anzahl von Atemzyklen zu unterbrechen.

Wird dem Patienten Atemgas während jeder zweiten, dritten oder n-ten Inspirationsphase zugeführt, so lassen sich erhebliche Mengen von Atemgas insbesondere bei solchen Patienten einsparen, die nur einen vergleichsweise geringen zusätzlichen Bedarf an Atemgas haben. Auch auf diese Weise sind kleine, energiesparende sowie geräuscharme Generatoren bei der Atemgasversorgung einsetzbar.

Bei der Vorrichtung der eingangs erwähnten Art besteht die erfindungsgemäße Lösung insbesondere darin, daß das Ventil(die Ventile)als Dosierventil ausgebildet ist(sind),welches(welche) zusammen mit dem Atemsensor an eine die Menge und/oder Strömungsgeschwindigkeit der Atemgaszufuhr während der Inspirationsphase eines Atemzyklus regelnde Steuereinrichtung an -geschlossen ist (sind).

Die Ventile der erfindungsgemäßen Vorrichtung sind als Dosierventile ausgebildet, über die nicht nur ein plötzlich einsetzender Atemgasschub oder -stoß mit etwa gleicher Volumenleistung pro Zeiteinheit abgegeben -, sondern über die vielmehr auch eine während der Inspirationsphase geregelte und beispielsweise pro Zeiteinheit vergleichsweise langsam ansteigende Gasmenge der Atemgasquelle entnommen werden kann. Dabei sind diese Dosierventile mit einer Steuereinrichtung verbunden, mit der der Verlauf des pro Zeiteinheit dem Patienten zugeführten Atemgas-Volumens an den Verlauf des pro Zeiteinheit vom Patienten eingeatmeten Luftvolumens über der Zeit zumindest in einem Teilbereich der Inspirationsphase angepaßt werden kann.

Eine einfache und vorteilhafte Ausführung gemäß der Erfindung sieht vor, daß je nach der dem Patienten während einer Inspirationsphase pro Zeiteinheit zuzuführenden Atemgasmenge in unterschiedlicher Anzahl öffen- und schließbare Dosierventile vorgesehen sind. Möglich ist aber auch, daß vorzugsweise nur ein einziges Dosierventil vorgesehen ist, dessen Austritts- oder Durchtrittsquerschnitt während der Atemgasabgabe verstellbar oder veränderbar ist.

Dabei sieht ein besonders vorteilhafter Vorschlag gemäß der Erfindung vor, daß das Dosierventil zwei relativ zueinander verstellbare und ineinander greifende Rohrabschnitte aufweist, daß eine dieser Rohrabschnitte eine oder mehrere Durchtrittsöffnungen hat, und daß durch Verstellbewegungen dieser Rohrabschnitte der Durchtrittsquerschnitt der Durchtrittsöffnung oder der Gesamt-Durchtrittsquerschnitt der Durchtrittsöffnungen veränderbar ist. Durch eine Dreh- oder Schiebebewegung der beiden relativ zueinander verstellbaren und ineinander greifenden Rohrabschnitte kann der Durchtrittsquerschnitt dieses Dosierventils entsprechend des pro Zeiteinheit gewünschten Volumens von Atemgas verändert werden. Zweckmäßigerweise sind dabei die beiden Rohrabschnitte des Dosierventils über zumindest einen Schrittmotor relativ zueinander verstellbar.

Eine Weiterbildung gemäß der Erfindung, für die selbstständiger Schutz beansprucht wird, sieht vor, daß in der Atemgas-Leitung zumindest eine pneumatische Ausdehnungskammer vorgesehen ist, die sich unter einem in ihre Innenhöhlung einströmenden Gasdruck ausdehnt und das aufgenommene Gasvolumen zeitverzögert wieder abgibt. Mit einer derartigen Ausdehnungskammer kann die Atemgaszufuhr an das dynamische Lungenvolumen sowie die Atemstromstärke (Atemzugvolumina pro Zeiteinheit) mit einfachen Mitteln angepaßt werden. Zweckmäßigerweise ist diese Ausdehnungskammer als pneumatischer Balgzylinder ausgebildet.

Weiterbildungen der Erfindung sind in weiteren Unteransprüchen aufgeführt. Nachstehend wird diese anhand vorteilhafter Ausführungsbeispiele in Verbindung mit den Figuren noch näher erläutert.

Es zeigt:

Fig. 1 ein aus zwei, im Querschnitt runden Rohrabschnitten bestehendes Dosierventil, mit einem etwa keilförmigen und durch Verschiebebewegungen der Rohrabschnitte öffen- und schließbaren Durchtrittsquerschnitt,

Fig. 2 ein aus ebenfalls zwei im Querschnitt runden Rohrabschnitten bestehendes Dosierventil, welches mehrere runde Durchtrittsöffnungen unterschiedlichen Durchmessers aufweist,

Fig. 3 ein Dosierventil, ähnlich dem aus Fig. 1, welches im wesentlichen aus zwei Rohrabschnitten rechteckigen Querschnitts besteht,

Fig. 4 ein ebenfalls aus zwei im Querschnitt rechteckigen Rohrabschnitten bestehendes Dosierventil,

Fig. 5 das Blockschema der Meß- und Steuereinrichtung einer Vorrichtung zur Zuführung von Atemgas, die über einen Schrittmotor beispielsweise eines der Ventile aus den Fig. 1 bis 4 öffnet und schließt,

Fig. 6 das Blockschema der Meß- und Steuereinrichtung einer Vorrichtung, bei der die Atemgaszufuhr über mehrere Magnetventile geregelt werden kann,

Fig. 7 eine als pneumatischer Balgzylinder ausgebildete Ausdehnungskammer in einer perspektivischen Darstellung,

Fig. 8 die Strömungscharakteristik einer, in ihrer Atemgas-Leitung eine Ausdehnungskammer aufweisenden Vorrichtung zur Zuführung von Atemgas und

Fig. 9 die mit Fig. 8 vergleichbare Strömungscharakteristik einer Vorrichtung ohne Ausdehnungskammer.

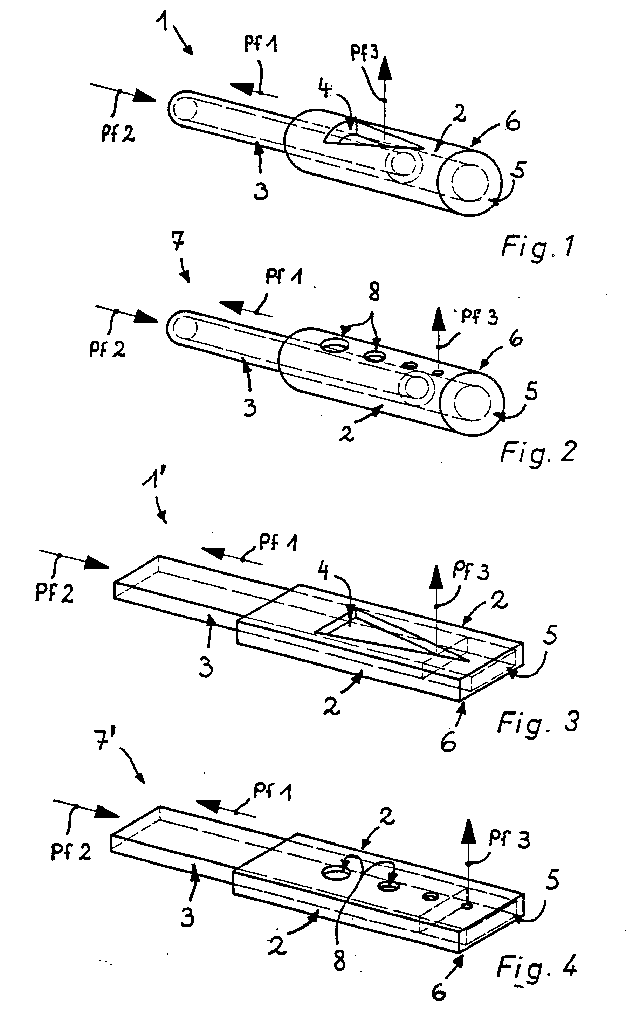

In den Fig. 1 bis 4 sind verschiedene Ausführungsformen des Ventils einer Vorrichtung zur Zuführung von Atemgas, insbesondere von Sauerstoff, zu einem selbst atmenden Patienten in perspektivischen Darstellungen gezeigt. Die ansonsten nicht weiter dargestellte Vorrichtung besteht im wesentlichen aus einer Atemgasquelle, eine Atemgaszuführeinrichtung, einem Inhalations-od. dgl. Atemsensor, einer Meßeinrichtung sowie dem mit der Meßeinrichtung und dem Atemsensor in Steuerverbindung stehenden Ventil.

Dabei ist die Atemgasquelle beispielsweise als ein kleiner Gasgenerator ausgebildet, der mit Hilfe von Zeolithen (Aluminiumsilikaten) die Luftkomponenten Stickstoff und Sauerstoff trennt sowie den Stickstoff unter Druck adsorbiert. Der dabei frei werdende Sauerstoff kann dem Patienten zur Anreicherung seiner Atemluft über ein Nasenröhrchen od. dgl. Atemgaszuführeinrichtung zugeführt werden. Um Sauerstoff einsparen und um den Gasgenerator klein und energiesparend ausbilden zu können, wird dem Patienten das Atemgas lediglich während der Inspirationsphasen seiner Atemzyklen zugeführt. Diese intermittierende Atemgaszufuhr wird u.a. mit Hilfe des Atemsensors erreicht, der mit einer die Atemzyklen registrierenden Meßeinrichtung sowie einem der hier dargestellten Ventile in Steuerverbindung steht.

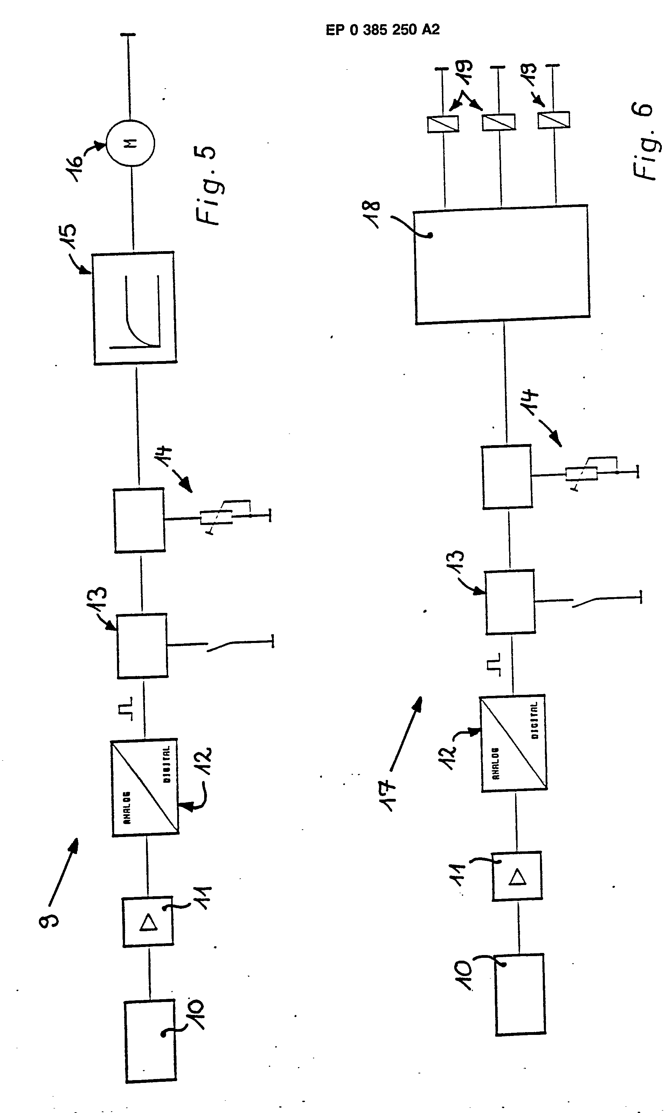

Um die Volumenleistung des dem Patienten zusätzlich zugeführten Atemgases über der Zeit an den Verlauf des pro Zeiteinheit vom Patienten eingeatmeten Luftvolumens über der Zeit zumindest in einem Teilbereich der Inspirationsphase anpassen und um dem Patienten eine ausreichende Atemgasmenge zuführen zu können, praktisch ohne daß dieser die Atemgaszufuhr als störend empfindet, sind die in den Fig. 1 bis 4 dargestellten Ventile erfindungsgemäß als Dosierventile ausgebildet, welche zusammen mit dem Atemsensor an eine die Atemgaszufuhr während der Inspirationsphase eines Atemzyklus regelnde Steuereinrichtung (vgl. beispielsweise Fig. 5) angeschlossen sind. Dabei ist der Austritts- oder Durchtrittsquerschnitt der in den Fig. 1 bis 4 dargestellten Dosierventile während der Atemgasabgabe verstellbar.

Dazu weist das in Fig. 1 dargestellte Dosierventil 1 zwei relativ zueinander verstellbare und ineinander greifende Rohrabschnitte 2, 3 auf, die jeweils einen runden Querschnitt haben und von denen der äußere Rohrabschnitt 2 mit einer Durchtrittsöffnung 4 versehen ist. Während der innere Rohrabschnitt 3 eine durchgehende, an beiden Endbereichen offene Innenhöhlung aufweist, ist der äußere Rohrabschnitt 2 an der Stirnseite 5 seines freien Endes 6 gasdicht verschlossen ausgebildet. Durch eine axiale Verschiebebewegung des inneren Rohrabschnitts 3 in Pfeilrichtung Pf 1 wird die in ihrem Durchtrittsquerschnitt etwa keilförmige Durchtrittsöffnung 4 des Dosierventils 1 zunehmend geöffnet. Das in Pfeilrichtung Pf 2 in den inneren Rohrabschnitt 3 einströmende Atemgas kann allein durch die Durchtrittsöffnung 4 in Pfeilrichtung Pf 3 aus dem Dosierventil 1 entweichen.

Da die Durchtrittsöffnung 4 in axialer Verschieberichtung Pf 1 der Rohrabschnitte 2, 3 eine größere Abmessung hat, wie in Umfangsrichtung, kann mit Hilfe des Dosierventils 1 über dessen Verstellweg hinweg die Atemgaszufuhr in ihrer Volumenleistung (V : t) gut eingeregelt werden. Wie Fig. 1 zeigt, nehmendie Abmessungen der Durchtrittsöffnung 4 in Umfangsrichtung der Rohrabschnitte 2, 3 zur völligen Offenstellung des Ventils 1 hin zu;

mit dem über den Verstellweg des Ventils 1 zur Offenstellung hin überproportional ansteigenden Durchtrittsquerschnitt der Durchtrittsöffnung 4 kann auch eine über den Verstellweg entsprechend ansteigende Volumenleistung der Atemgaszufuhr erreicht werden.

Die hier dargestellte Ausführungsform eines Dosierventils 1 und insbesondere die keilförmige Ausgestaltung von dessen Durchtrittsöffnung 4 begünstigt somit die Anpassung des Verlaufs des pro Zeiteinheit zugeführten Atemgas-Volumens über der Zeit an den Verlauf des pro Zeiteinheit eingeatmeten Luftvolumens über der Zeit, der nämlich zu Beginn der Inspirationsphase zunächst schwach und etwa in der Mitte der Inspirationsphase des Patienten stärker ansteigt. Durch die Anpassung der Atemgaszufuhr an den natürlichen Verlauf des Atemvorgangs wird praktisch vermieden, daß dem Patienten das Atemgas in Gasstössen oder -schüben zugeführt wird, die ihn, insbesondere in Ruhephasen, erheblich stören könnten.

Fig. 2 zeigt ein Dosierventil 7, das mit Ausnahme der Durchtrittsöffnung in seinem Aufbau weitgehend dem in Fig. 1 dargestellten Dosierventil 1 entspricht. Statt einer etwa keilförmigen Durchtrittsöffnung 4 weist das Dosierventil 7 aus Fig. 2 jedoch mehrere, runde Durchtrittsöffnungen 8 auf, deren Durchmesser in axialer Verschieberichtung Pf 1 zur völligen Offenstellung des Dosierventils 7 hin jeweils zunehmen. Somit wird auch bei dem Dosierventil 7 ein über dessen Verschiebeweg zur völligen Offenstellung hin überproportional ansteigender Durchtrittsquerschnitt erzielt.

Die Figuren 3 und 4 zeigen Dosierventile 1`, 7`, die in ihrem Aufbau weitgehend den in Fig. 1 und 2 dargestellten Dosierventilen 1, 7 entsprechen. Jedoch weisen die Rohrabschnitte 2, 3 der Dosierventile 1`, 7` einen etwa viereckigen Querschnitt auf.

Die inneren und äußeren Rohrabschnitte 2, 3 der Dosierventile 1, 1`, 7 und 7` sind beispielsweise jeweils zumindest über einen - hier nicht dargestellten - Schrittmotor relativ zueinander verstellbar, der in bekannter Weise an diesen Rohrabschnitten 2, 3 angreift.

Statt den hier dargestellten Ventilen 1, 1`, 7 und 7` ist beispielsweise auch eine Ausführungsform möglich, bei der je nach der dem Patienten während einer Inspirationsphase pro Zeiteinheit zuzuführenden Atemgasmenge (Atemgas-Volumen) in unterschiedlicher Anzahl öffen- und schließbare Dosierventile, insbesondere Magnetventile, vorgesehen sind.

In Fig. 5 ist das Blockschema der Meß- und Steuereinrichtung 9 einer Vorrichtung zur Zuführung von Atemgas dargestellt. Dabei wird über einen Inhalations- od. dgl. Atemsensor 10 beispielsweise der Beginn der Inspirationsphase des Patienten während seiner Atemzyklen registriert. Ein solcher Sensor 10 kann beispielsweise als Temperatursensor ausgebildet sein, der, etwa in der Atemgaszuführeinrichtung, so angeordnet ist, daß er sowohl von dem zugeführten Atemgas als auch von der ausgeatmeten Luft wechselweise beaufschlagbar ist. Da das zugeführte Atemgas und die ausgestossene, verbrauchte Atemluft wesentliche Temperaturunterschiede aufweisen, ist der Wechsel zwischen Einatmung und Ausatmung mit Hilfe des Atemsensors 10 deutlich meßbar.

Die vom Atemsensor 10 kommenden, analogen Signale werden in einem Analogverstärker 11 verstärkt und anschließend in einem Analog-/Digital-Wandler 12 in digitale Signale umgewandelt. In einem Rechner 13 der Meß- und Steuereinrichtung 9 wird aus den, mehreren Atemzyklen entsprechenden Signalen des Atemsensors 10 der nahezu aktuelle Abstand zwischen den Atemzyklen errechnet. Dieser Rechner 13 erlaubt es somit, den Startpunkt der Atemgaszufuhr in einer beliebigen Phase des Atemzyklus auszulösen und/oder die Impulsfolge der Atemgaszufuhr um eine beliebige Anzahl von Atemzyklen zu unterbrechen. Dabei ist in den Rechner 13 ein Korrekturfaktor eingebbar oder einstellbar, der der Zeitverzögerung zwischen dem Erhalt eines Signals vom Atemsensor 10 und dem Startpunkt der Atemgasabgabe etwa entspricht. Somit kann der Startpunkt jeder Atemgaszufuhr jeweils an den Beginn der Inspirationsphasen des Patienten angenähert werden. Über ein Potentiometer 14 kann auch die Pulslänge der Atemgaszufuhr während einer Inspirationsphase eingeregelt und beispielsweise so eingestellt werden, daß jede Atemgaszufuhr während der Inspirationsphase mit zeitlichem Abstand vor der nachfolgenden Exspirationsphase des Atemzyklus beendet wird.

Mit Hilfe der Rampensteuerung 15 der in Fig. 5 dargestellten Meß- und Steuereinrichtung 9 ist der Öffnungs- und Schließvorgang des Dosierventils über den Schrittmotor 16 in einer Rampenfunktion steuerbar. Durch den in einer Rampenfunktion gesteuerten Schrittmotor kann der Öffnung- und Schließvorgang des Dosierventils so eingeregelt werden, daß die Anpassung der Atemgaszufuhr an den natürlichen Atemvorgang noch begünstigt wird. Wie die gesamte Vorrichtung zur Zuführung von Atemgas, so wird auch deren Meß- und Steuereinrichtung 9 über das Stromnetz betrieben. Dabei wird für die Meß- und Steuereinrichtung der 220 V/50 Hz-Wechselstrom in eine Gleichspannung umgewandelt.

Die in Fig. 5 dargestellte Meß- und Steuereinrichtung 9 kann vorteilhaft beispielsweise mit einem der in den Fig. 1 bis 4 dargestellten Dosierventile 1, 1`, 7 und 7` zusammenwirken.

In Fig. 6 ist eine gegenüber der in Fig. 5 dargestellten Ausführungsform geringfügig abgewandelte Meß- und Steuereinrichtung 17 dargestellt, die statt einer Rampensteuerung 15 (vgl. Fig. 5) eine Magnet-Ventil-Steuerung 18 für mehrere, als Magnetventile ausgebildete Dosierventile 19 aufweist.

Die Dosierventile 19 sind über die Magnet-Ventil-Steuerung 18 je nach der dem Patienten während einer Inspirationsphase pro Zeiteinheit zuzuführenden Atemgasmenge in unterschiedlicher Anzahl öffen- und schließbar.

Da die Meß- und Steuereinrichtungen 9, 17 aus den vom Atemsensor 10 kommenden Signalen praktisch den aktuellen Abstand zwischen den Atemzyklen und ihren Inspirationsphasen errechnen, wird über diese Steuereinrichtungen die Atemgaszufuhr weitgehend auch an dem momentanen Luft- und Sauerstoff-Bedarf des Patienten angepaßt.

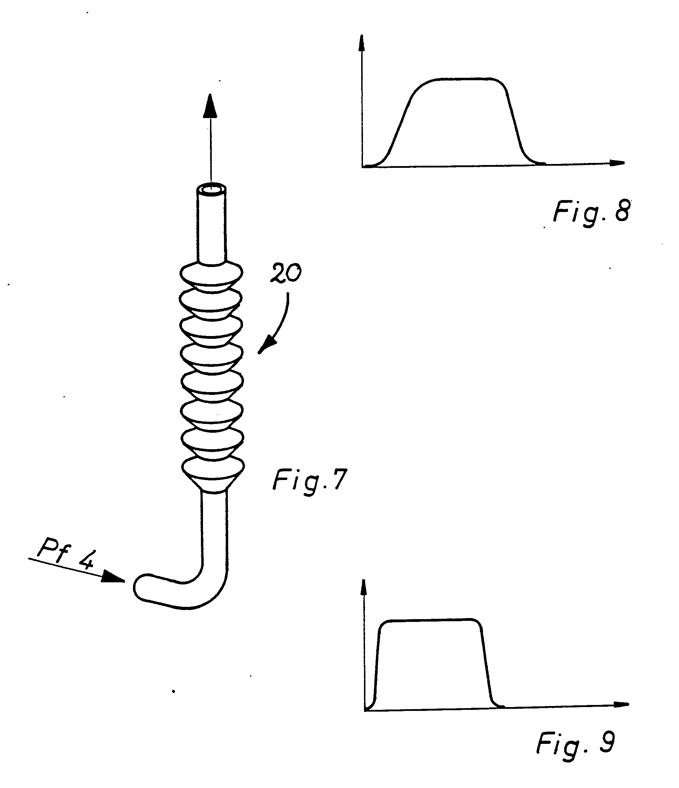

Fig. 7 zeigt in einer perspektivischen Darstellung eine pneumatische Ausdehnungskammer 20, die hier als pneumatischer Balgzylinder ausgebildet ist. Die Ausdehnungskammer 20 ist in der Atemgas-Leitung der Vorrichtung zur Zuführung von Atemgas zweckmäßigerweise in Strömungsrichtung hinter dem Dosierventil oder den Dosierventilen angeordnet. Die Ausdehnungskammer 20 kann jedoch auch für sich alleine beispielsweise ohne die hier dargestellten Ventile zur weitgehenden Anpassung der Atemgaszufuhr verwendet werden. Die pneumatische und mit ihrer Längsachse vorzugsweise vertikal angeordnete Ausdehnungskammer 20 dehnt sich bei einer Atemgaszufuhr in Pfeilrichtung Pf4 zunächst in ihrer Länge aus, wodurch das in der Ausdehnungskammer 20 gespeicherte oder speicherbare Gasvolumen zunimmt. Entsprechend hat der in Strömungsrichtung hinter der Ausdehnungskammer gemessene Verlauf des pro Zeiteinheit zugeführten Atemgas-Volumens in dem, dem Startpunkt der Atemgaszufuhr unmittelbar folgenden Zeitabschnitt eine vergleichsweise schwächere Steigung, die dem natürlichen Atemvorgang besser entspricht und vom Patienten daher auch weniger als störend empfunden wird. Hat die Ausdehnungskammer unter weiterer Atemgas-zufuhr ihr maximales Ausdehnungsvolumen er-reicht, so strömt auch das Atemgas weitgehend ungehindert durch die Ausdehnungskammer 20.

Nach Beendigung der Atemgaszufuhr während einer Inspirations phase des Patienten zieht die Ausdehnungskammer 20 sich, bedingt durch ihre Elastizität und ihr Eigengewicht, wieder zusammen; dadurch wird dem Patienten zusätzlich Atemgas zugeführt und das Absinken des Verlaufs des pro Zeiteinheit zugeführten Atemgas-Volumens gedämpft und verzögert.

In den Fig. 8 und 9 ist die Strömungscharakteristik einer, mit einer Ausdehnungskammer 20 versehenen Vorrichtung zur Zuführung von Atemgas (vgl. Fig. 8) im Vergleich zu einer Vorrichtung gezeigt, die weder eine Ausdehnungskammer 20, noch irgendwelche erfindungsgemäß ausgebildeten Dosierventile aufweist.

Deutlich erkennbar ist, daß die Atemgaszufuhr einer Vorrichtung mit Ausdehnungskammer im Bereich der Start- und Endpunkte der Gaszufuhr (vgl. Fig. 8) mit einer geringeren Steigung zunimmt und abfällt und daher dem natürlichen Atemvorgang besser entspricht. Demgegenüber stellt die Strömungscharakteristik einer ebenfalls intermittierend Atemgas zuführende Vorrichtung, deren Ventil lediglich geöffnet und geschlossen, nicht aber gesteuert werden kann, und die in ihrer Atemgasleitung keine Ausdehnungskammer 20 aufweist, einen Atemgasstoß oder -schub dar, der vom Patienten häufig als störend empfunden wird (vgl. Fig. 9).

Die pneumatische Ausdehnungskammer 20 kann - wie hier - als Balgen, beispielsweise aber auch als Ballon, oder Faltenschlauch aus weichem, dehnbarem Gummi- oder Kunststoffmaterial ausgebildet sein. Um die Strömungscharakteristik einer solchen Ausdehnungskammer auch verändern zu können, kann es vorteilhaft sein, wenn die durch den Gasdruck erzielbare Ausdehnung der Ausdehnungskammer begrenzbar und vorzugsweise auch einstell- und festlegbar ist. Dazu könnte beispielsweise die als pneumatischer Balgzylinder ausgebildete Ausdehnungskammer 20 im Inneren von zwei ineinander greifenden und an ihren freien Endbereichen einen Stützboden aufweisenden Röhren angeordnet sein, die zur Begrenzung der durch den Gasdruck erzielbaren Ausdehnung der Ausdehnungskammer etwa mit Hilfe zumindest einer Stellschraube in ihrer Relativposition zueinander verstellbar sind.

Die Erfindung betrifft eine Vorrichtung, die den Gasstrahl so langsam einsetzen läßt, daß der spontan atmende Patient den Vorgang toleriert.

Um mit der intermittierenden Gaszufuhr möglichst viel Gas einsparen zu können, ist es wichtig, den Gasimpuls möglichst zu Beginn der Inspirationsphase wirksam werden zu lassen. Löst eine im Anfang der Inspirationsphase erzeugtes Triggersignal den Gasimpuls aus, so bewirkt das langsame Einsetzen eine Verschiebung in Richtung Inspirationsende. Die Erfindung betrifft daher auch eine Vorrichtung, die aus mehreren spontanen Atemzügen den Abstand zwischen den Atemzyklen elektronisch errechnet und es daher erlaubt, den Impuls in einer beliebigen Phase des Atemzyklus auszulösen und/oder die Impulsfolge um eine beliebige Anzahl von Atemzyklen zu unterbrechen.

Um die Strömungscharakteristik der zusätzli-chen Atemgaszufuhr an den natürlichen Atemvorgang eines Patienten anpassen zu können, werden beispielsweise Magnetventile mit keilförmig gestal-teter Auslaßöffnung, Magnetventile mit nacheinan-

der zu öffnenden Auslaßbohrungen, mehrere Magnetventile, die nacheinander eingeschaltet werden und so nacheinander Teilströmungen zuschalten oder ein entsprechendes Schiebeventil vorgeschlagen.

Dabei kann die Steuerung der Ventile beispielsweise mit einem Schrittmotor mit vorgeschaltetem Impulsgeber erfolgen, wobei Form und Dauer der Gasströmung entweder durch Formgebung der Ventilöffnungen oder durch die Impulsfolge am Schrittmotor gestaltet werden können.

Die Steuerung der Ventile kann aber auch über den Rechner einer Meß- und Steuereinrichtung oder - bei einem Schieberventil - durch einen pneumatischen Antrieb unter Benutzung des Luftverdichters im Sauerstoffkonzentrator oder einer anderen Sauerstoffquelle der Vorrichtung zur Zuführung von Atemgas erfolgen. Die der Errechnung des nächsten Zykluseinsatzes sowie zur Steuerung der Vorrichtung dienende Atemsensorkontrolle der Atemfrequenz kann wahlweise beispielsweise über Temperatur, Druck,oder Strömung erfolgen.

Es sei noch angemerkt, daß der Stellvorgang der in den Fig. 1 - 4 dargestellten Ventile 1, 1`, 7, 7` auch durch das während den Inspirationsphasen durch diese Ventile jeweils durchströmende Atemgas bewirkt und daher auf Schrittmotoren und dergleichen auch verzichtet werden kann. Zweckmäßigerweise weisen die Ventile bei einer solchen Ausführungsform jeweils zumindest ein insbesondere als Feder ausgebildetes Rückstellelement auf, welches das entsprechende Ventil beispielsweise bei einer während den Exspirationsphasen unterbrochenen Atemgaszufuhr wieder in seine Schließstellung bringt.

Alle vorbeschriebenen oder in den Ansprüchen aufgeführten Einzelmerkmale können einzeln oder in beliebiger Kombination miteinander erfindungswesentlich sein.

**Ansprüche**

1. Verfahren zur Zuführung von mit Sauerstoff angereichertem, zusätzlichem Atemgas von einer Atemgasquelle zu einem spontan atmenden Patienten, wobei das Atemgas dem Patienten während der eigenen Aktivierung seiner Lunge beim Einatmen (Inspirationsphase eines Atemzyklus) zugeführt wird, **dadurch gekennzeichnet,** daß der Verlauf des pro Zeiteinheit dem Patienten zugeführten Atemgas-Volumens an den Verlauf des pro Zeiteinheit vom Patienten aktiv eingeatmeten Luftvolumens über der Zeit zumindest in einem Teilbereich der Inspirationsphase etwa angepaßt wird.

2. Verfahren nach Anspruch 1, dadurch gekennzeichnet, daß das pro Zeiteinheit zugeführte Atemgas-Volumen über der Zeit während der Inspirationsphase zunächst zunimmt und vorzugsweise etwa in gleichem Maß wie das vom Patienten eingeatmete Luftvolumen über der Zeit gesteigert wird.

3. Verfahren nach Anspruch 1 oder 2, dadurch gekennzeichnet, daß das pro Zeiteinheit zugeführte Atemgas-Volumen über der Zeit während der Inspirationsphase auf etwa die Werte des vom Patienten selbst eingeatmeten Luft-Volumens über der Zeit eingeregelt wird.

4. Verfahren nach einem oder mehreren der Ansprüche 1 bis 3, dadurch gekennzeichnet, daß der Startpunkt der von dem Beginn der Inspirationsphase ausgelösten Atemgaszufuhr eingestellt und vorzugsweise jeweils an den Beginn der Inspirationsphase angenähert wird.

5. Verfahren nach einem oder mehreren der Ansprüche 1 bis 4, dadurch gekennzeichnet, daß von einer Steuereinrichtung aus den von einem Atemsensor kommenden Signalen der Abstand zwischen den Inspirationsphasen der spontanen Atemzyklen errechnet und mit Hilfe dieses Rechenergebnisses der Startpunkt der Atemgasabgaben vorzugsweise jeweils dem Beginn der Inspirationsphasen angenähert wird.

6. Verfahren nach einem oder mehreren der Ansprüche 1 bis 5, dadurch gekennzeichnet, daß das dem Patienten pro Zeiteinheit zugeführte Volumen von zusätzlichem Atemgas auf einen Höchstbetrag begrenzt wird, der vorzugsweise gleich oder kleiner ist als das vom Patienten maximal pro Zeiteinheit eingeatmete Luft-Volumen.

7. Verfahren nach einem oder mehreren der Ansprüche 1 bis 6, dadurch gekennzeichnet, daß jede Atemgaszufuhr während der Inspirationsphase mit zeitlichem Abstand vor der nachfolgenden Exspirationsphase des Atemzyklus beendet wird.

8. Verfahren nach einem oder mehreren der Ansprüche 1 bis 7, dadurch gekennzeichnet, daß dem Patienten Atemgas während jeder zweiten, dritten oder n-ten Inspirationsphase zugeführt wird.

9. Vorrichtung zur Zuführung von zusätzlichem Atemgas zu einem spontan bzw. selbst atmenden Patienten, insbesondere zur Durchführung des Verfahrens nach einem oder mehreren der Ansprüche 1 bis 8, mit einer Atemgasquelle, einer Atemgaszuführeinrichtung, einem Inhalations- oder dergleichen Atemsensor, einer Meßeinrichtung sowie zumindest einem, mit dem Atemsensor und der Meßeinrichtung in Steuerverbindung stehenden Ventil, dadurch gekennzeichnet, daß das Ventil (die Ventile) als Dosierventil ausgebildet ist (sind), welches (welche) zusammen mit dem Atemsensor (10) an eine die Menge und/oder Strömungsgeschwindigkeit der Atemgaszufuhr während der Inspirationsphase regelnde Steuereinrichtung (9, 17) angeschlossen ist (sind).

10. Vorrichtung nach Anspruch 9, dadurch gekennzeichnet, daß je nach der dem Patienten während einer Inspirationsphase pro Zeiteinheit zuzuführenden Atemgasmenge in unterschiedlicher Anzahl öffen- und schließbare Dosierventile (19) vorgesehen sind.

11. Vorrichtung nach Anspruch 9 oder 10 dadurch gekennzeichnet, daß vorzugsweise nur ein einziges Dosierventil (1, 1`, 7, 7`) vorgesehen ist, dessen Austritts- oder Durchtrittsquerschnitt während der Atemgasabgabe verstellbar ist.

12. Vorrichtung nach einem oder mehreren der Ansprüche 9 bis 11, dadurch gekennzeichnet, daß das Dosierventil (1, 1`, 7, 7`) zwei relativ zueinander verstellbare und ineinander greifende Rohrabschnitte (2,3) aufweist, daß einer dieser Rohrabschnitte (2, 3) eine oder mehrere Durchtrittsöffnungen (4, 8) hat, und daß durch Verstellbewegungen dieser Rohrabschnitte (2, 3) der Durchtrittsquerschnitt der Durchtrittsöffnung (4) oder der Gesamtdurchtrittsquerschnitt der Durchtrittsöffnungen (8) veränderbar ist.

13. Vorrichtung nach einem oder mehreren der Ansprüche 9 bis 12, dadurch gekennzeichnet, daß die auf einem Rohrabschnitt (2) des Dosierventils (1, 1`) vorgesehene Durchtrittsöffnung (4) in axialer Verschieberichtung der Rohrabschnitte (2, 3) eine größere Abmessung hat wie in Umfangsrichtung.

14. Vorrichtung nach einem oder mehreren der Ansprüche 9 bis 13, dadurch gekennzeichnet, daß die Abmessungen der Durchtrittsöffnung (4) in Umfangsrichtung der Rohrabschnitte (2, 3) zur völligen Offenstellung des Ventils (1, 1`) hin zunehmen.

15. Vorrichtung nach einem oder mehreren der Ansprüche 9 bis 14, dadurch gekennzeichnet, daß die Durchtrittsöffnung (4) etwa keilförmig ausgebildet ist.

16. Vorrichtung nach einem oder mehreren der Ansprüche 9 bis 15, dadurch gekennzeichnet, daß das Dosierventil (7, 7`) mehrere Durchtrittsöffnungen (8) aufweist, deren Durchmesser in axialer Verschieberichtung (Pf 1) zur völligen Offenstellung des Dosierventils (7, 7`) hin Vorzugsweise jeweils zunehmen.

17. Vorrichtung nach einem oder mehreren der Ansprüche 9 bis 16, dadurch gekennzeichnet, daß die Rohrabschnitte (2, 3) des Dosierventils (der Dosierventile) (1, 1`, 7, 7`) einen runden oder viereckigen Querschnitt aufweisen.

18. Vorrichtung nach einem oder mehreren der Ansprüche 9 bis 17, dadurch gekennzeichnet, daß die Durchtrittsöffnung(en) auf dem äußeren Rohrabschnitt (2) des Dosierventils (1, 1`, 7, 7`) vorgesehen ist (sind), und daß das freie Ende (6) dieses Rohrabschnitts (2) vorzugsweise gasdicht verschlossen ist.

19. Vorrichtung nach einem oder mehreren der Ansprüche 9 bis 18, dadurch gekennzeichnet, daß die beiden Rohrabschnitte (2,3) des Dosierventils (1, 1`, 7, 7`) über zumindest einen Schrittmotor (16) relativ zueinander verstellbar sind.

20. Vorrichtung nach einem oder mehreren der Ansprüche 1 bis 19, dadurch gekennzeichnet,daß der Öffnungs- und/oder Schließvorgang des Dosierventils (der Dosierventile) über den Schrittmotor (die Schrittmotoren) (16) in einer Rampenfunktion steuerbar ist.

21. Vorrichtung, insbesondere nach einem der Ansprüche 9 bis 20, dadurch gekennzeichnet, daß in der Atemgasleitung zumindest eine pneumatische Ausdehnungskammer (20) vorgesehen ist, die sich unter einem in ihre Innenhöhlung einströmenden Gasdruck ausdehnt und das aufgenommene Gasvolumen zeitverzögert wieder abgibt.

22. Vorrichtung nach Anspruch 21, dadurch gekennzeichnet, daß die Ausdehnungskammer (20) als pneumatischer Balgzylinder ausgebildet ist.

23. Vorrichtung nach Anspruch 21 oder 22, dadurch gekennzeichnet, daß die durch den Gasdruck erzielbare Ausdehnung der Ausdehnungskammer (20) begrenzbar und vorzugsweise auch einstell- und festlegbar ist.

24. Vorrichtung nach einem oder mehreren der Ansprüche 21 bis 23, dadurch gekennzeichnet, daß die Ausdehnungskammer in der Atemgas-Leitung in Strömungsrichtung hinter dem Dosierventil (den Dosierventilen) angeordnet ist.

25. Vorrichtung nach einem oder mehreren der Ansprüche 9 bis 24, dadurch gekennzeichnet, daß die Steuereinrichtung (9, 17) einen Rechner zur Berechnung des Abstandes zwischen den Inspirationsphasen der Atemzyklen aus den vom Atemsensor (10) kommenden Signalen sowie zur Einstellung des Startpunktes der Atemgasabgabe während den Inspirationsphasen hat.

26. Vorrichtung nach einem oder mehreren der Ansprüche 9 bis 25, dadurch gekennzeichnet, daß in den Rechner zur Auslösung der Atemgasabgabe ein Korrekturfaktor eingebbar oder einstellbar ist, der der Zeitverzögerung zwischen dem Erhalt eines Signals vom Atemsensor (10) und dem Startpunkt der Atemgasabgabe etwa entspricht.

1

Pf 1

Pf 3

4

2

6

Pf 2

3

5

*Fig. 1*

7

Pf 1

8

Pf 3

6

Pf 2

3

5

2

*Fig. 2*

1'

Pf 1

4

Pf 3

2

Pf 2

3

5

2

6

*Fig. 3*

7'

Pf 1

2

Pf 3

5

Pf 2

3

2

8

6

*Fig. 4*

Fig. 5

Fig. 6

Fig. 8

20

Fig. 7

Pf 4

Fig. 9